# EUROPEAN PATENT SPECIFICATION

(11) **EP 3 305 256 B1**
(45) Date of publication and mention of the grant of the patent: **06.01.2021**
(21) Application number: 16799395.5
(22) Date of filing: 26.05.2016
(51) Int. Cl.: A61F 5/453

(54) **HYGIENIC PROTECTOR FOR MALE URINATION**
HYGIENISCHE SCHUTZABDECKUNG BEIM MÄNNLICHEN URINIEREN
PROTECTION HYGIÉNIQUE POUR LA MICTION MASCULINE

(30) Priority: 27.05.2015 ES 201500374 U
(43) Date of publication of application: 11.04.2018
(73) Proprietor: PROYECTOS A.M.J. 2017, S.L., 12530 Burriana Castellón (ES)
(72) Inventor: LLOPIS AMOROS, Javier, 12530 Burriana (Castellón) (ES)
(74) Representative: De la Fuente Fernandez, Dionisio
(86) International application number: PCT/ES2016/000063
(87) International publication number: WO 2016/189170

(56) References cited:
- WO-A1-02/38089
- DE-A1- 4 343 789
- DE-A1-102013 105 862
- GB-A- 2 512 905
- JP-A- 2009 095 672
- US-A- 5 566 400
- US-A1- 2006 090 250

## Description

The object of the present invention is a device as described in the claims by way of cover of the penis to ensure hygiene and cleaning during male urination, and behind it.

### BACKGROUND OF THE INVENTION

All men are facing different problems during urination, among which it can quote, dirty hands and need to wash them before or get dirty hands during the action, and not having the means to do so, or those droplets that dirty clothes inside or the pants after urinating or splashes occurring and causing so many marital disagreements.

The hygienic protector for male urination is a device easy manufacturing and low cost which solves the above drawbacks and contributes to the hygiene and cleanliness of the users and services.

The patent application US2006 / 0090250 shows a urinary urination device constituted by a simple sheet of flexible material, substantially flat, sensibly receptive to water, which can provide a guide for the urine to flow from the urethra to a receptacle opening located by below. The manufacturing material can be eliminated in sewer systems without clogging or damaging the systems. The preferred material of the invention are nonwoven, lightweight sheets similar to conventional toilet paper and which have been modified to not be entirely wet in the urine. A preferred material is a nonwoven, wet, biodegradable web material similar to the exemplified in US 4,755,421. The device of the invention can also be formed from a thin and lightweight paper which can be coated on the outside to be at least temporarily impermeable, but which is sufficiently biodegradable to allow it to empty to dispose of the urinary support once Has been used. The problem presented by the patent application US2006 / 0090250 is that the provision of a flat sheet of flexible material to direct the urine does not ensure that urination is oriented correctly, as it can flow through any part of the sheet, since it depends a lot on folding the sheet of material, in such a way that it does not guarantee the user a total safety at the time of urinating, so that urination is safe it is necessary that the device present a form such as to allow orienting the flow of urine from the user's penis, this is achieved by means of the present invention, since it consists of a tube of cylindrical or frustoconical shape that adapts to the end of the penis and said end intended to be in contact with the glans is folded towards the interior of the tube and the portion of the tube folded inward has a plurality of circumferential folds ensuring that the direction of the urine either by a single route and this is not expelled by any part, in addition the present invention comprises one or more fins for its manipulation by the user which increases its reliability.

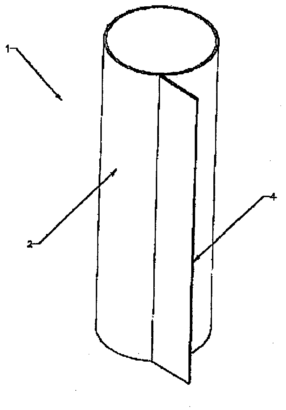

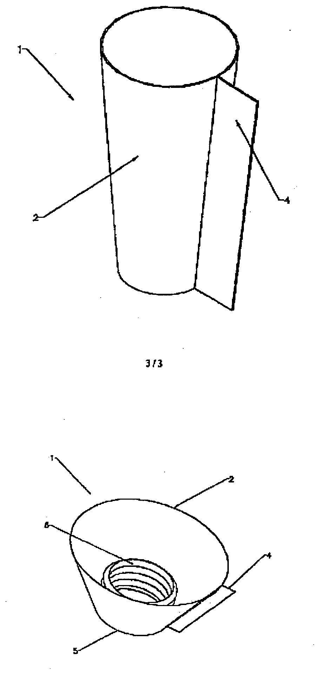

The German patent DE43433789 disclose a hygienic protector for male urination characterised that it consists of a hollow tube which is cylindrical (Fig. 1 and 2) of an absorbent and elastic textile material, such as paper and that it has one or more fins (15, 15a) arranged on the outer surface of the referred hollow tube,

The patent application DE102013105862 reveal fundamentally a micturition aid having a tubular body (1) formed from one or more leaves and having an inlet opening (2) at an inlet end (12) and an outlet opening (3) at an outlet end (13), wherein the inlet end (12) and the outlet end (12) 13) are arranged at longitudinally opposite ends of the tubular body (1), characterized in that a first sheet edge region (41) is connected to a second sheet edge region (51) by means of a longitudinal embossing seam (6), further the tubular body is formed from a single sheet and that the longitudinal embossing seam connects a first sheet edge region with a secondsheet edge region of the sheet.

The document WO 0238089 relates to an apparatus for aiding a woman in urinating in a standing position. More particularly, this invention relates to a portable, foldable, disposable, biodegradable and leakproof sanitary product that conforms comfortably to the body and directs the urine away from the body to exterior disposal, allowing women to urinate without sitting down. Its light, compact, collapse/emerge folding design is convenient and discrete, and the product has a disposable design intended to be used only once. The apparatus according to the invention is characterized in that it is foldable and leakproof and has a flat-design with two layers, which are either natural folded extensions of each other or simply affixed to each other on the sides.

The japanese patent JP2009095672 The urinary directional device for improving the directional urination of a male user is provided. The device comprises a conduit for the passage of urine having a proximal end adapted for fitting the penis of the user and having a proximal aperture to enable the passage of urine into the conduit and a distal end having a distal aperture to enable the passage of urine from the conduit, the device having an interior surface and an exterior surface. The interior surface providing one or more of a cleansing, an anti-bacterial, an antiseptic or an anti-fungal function whereby the penis of the user can be cleansed by wiping on the interior surface of the device after urination.

### DESCRIPTION OF THE INVENTION

The hygienic protector for male urination of the present invention is described in the claims.

### BRIEF DESCRIPTION OF THE FIGURES

Figure 1; shows a view in perspective of a first priority realization of the present disclosure.
Figure 2; shows a view in perspective of a second priority realization of the present disclosure.
Figure 3: shows a view in perspective of a third preferred realization of the present disclosure.

### PREFERRED EMBODIMENT OF THE INVENTION

Figure 1 shows a first priority realization of the hygienic protector for urination male, which consists in a Hollow tube, preferably approximately cylindrical shape, absorbent, elastic textile material, whose surface outside has one or more fins to facilitate handling.

Figure 2 shows a second preferred realization of the present disclosure which consists in a hollow tube, preferably approximately Pan shape, of a materialabsorbent and elastic textile, preferably paper, whose outer surface has one or more fins to facilitate its manipulation.

Figure 3 shows a third preferred realization of the present disclosure, which consists in a preferably hollow tube of Pan shape (2), an absorbent textile material and elastic, preferably paper, whose outer surface has one or more fins to facilitate handling. In addition, the end (5) destined to be in contact with the Glans is folded towards the inside of the pipe concerned and the portion of the tube folded inward can have a plurality of circumferential pleats. (6)

As a design option, the surface of the pipe (2) hole has a plurality of pleats, not shown in the figure, uniformly distributed to facilitate their adaptation to different sizes of penises.

The way to use the hygienic protector for male urination, which can come in boxes, is take one from the box, extend the folded portion of tube to inside, grab it by the flaps or stretch it with the fingers, insert the penis until the Glans protrudes approximately 1 cm, urinate, partially remove the invention, shaken to collect the last drops, remove it and dry the tip of the Glans and finally throw away it to the litter bin.

In order to improve the cleaning, remove odors and prevent diseases, the inside surface of the hollow tube may incorporate a cleansing cream for skin and mucous membranes or a disinfectant cream.

The advantages of the present invention include, but are not limited, it is not necessary to wash the hands before or after urination, being absorbent paper, collects the last drops so stains do not originate in the underwear or pants and if the penis during urination is completely inside the cover, reduces splashes with which reduces the number of times that wash services and removed almost completely, the odor-causing.

## Claims

1. Hygienic Protector for male urination that comprise a hollow tube (2) of an absorbent and elastic textile material that it has one or more fins (4) arranged on the outer surface of the referred hollow tube (2) wherein the surface of the tube (2) has of a plurality of evenly distributed longitudinal folds **characterised in that** the end (5) to be in contact with the glans is folded towards the inside of the referred tube (2) and the portion tube bending inward it has a plurality of circumferential folds (6).

2. Hygienic Protector for male urination, according to claim 1, **characterized in that** the tube (2) is approximately cylindrical shaped.

3. Hygienic Protector for male urination, according to the claim 1, **characterized in that** the tube (2) is truncated cone shape.

4. Hygienic Protector for male urination, according to any of the previous claims, **characterized in that** the textile material is paper.

5. Hygienic Protector for male urination, according to any of the preceding claims, **characterized in that** the inside of the tube (2) surface incorporates a cleansing cream for skin and mucous membranes.

6. Hygienic Protector for male urination, according to any of the above claims, **characterized in that** the inner surface of the tube (2) incorporates a disinfectant cream.

## Patentansprüche

1. Hygienische Schutzabdeckung zum männlichen Urinieren, die ein Hohlrohr (2) aus einem saugfähigen und elastischen Textilmaterial umfasst, das eine oder mehrere Lamellen (4) aufweist, die auf der Außenfläche des genannten Hohlrohrs (2) angeordnet sind, wobei die Oberfläche des Rohres (2) eine Vielzahl von gleichmäßig verteilten Längsfalten aufweist, **dadurch gekennzeichnet, dass** das Ende (5), das mit der Eichel in Kontakt kommen soll, zur Innenseite des genannten Rohrs (2) hin gefaltet ist und der Rohrabschnitt, der nach innen gebogen ist, eine Vielzahl von Umfangsfalten (6) aufweist.

2. Hygienische Schutzabdeckung zum männlichen Urinieren nach Anspruch 1, **dadurch gekennzeichnet, dass** das Rohr (2) annähernd zylindrisch geformt ist.

3. Hygienische Schutzabdeckung zum männlichen Urinieren nach Anspruch 1, **dadurch gekennzeichnet, dass** das Rohr (2) kegelstumpfförmig ist.

4. Hygienische Schutzabdeckung zum männlichen Urinieren nach einem der vorhergehenden Ansprüche, **dadurch gekennzeichnet, dass** das Textilmaterial Papier ist.

5. Hygienische Schutzabdeckung zum männlichen Urinieren nach einem der vorangehenden Ansprüche, **dadurch gekennzeichnet, dass** die Innenseite der Oberfläche des Rohres (2) eine Reinigungscreme für Haut und Schleimhäute enthält.

6. Hygienische Schutzabdeckung zum männlichen Urinieren nach einem der obigen Ansprüche, **dadurch gekennzeichnet, dass** die Innenfläche des Rohres (2) eine Desinfektionscreme enthält.

## Revendications

1. Protecteur hygiénique pour la miction masculine qui comprend un tube creux (2) en une matière textile absorbante et élastique qui a une ou plusieurs ailettes (4) disposées sur la surface externe du tube creux (2) référé dans lequel la surface du tube (2) a une pluralité de plis longitudinaux uniformément répartis **caractérisé en ce que** l'extrémité (5) destinée à être en contact avec le gland est repliée vers l'intérieur du tube (2) référé et la portion de tube se courbant vers l'intérieur a une pluralité de plis circonférentiels (6).

2. Protecteur hygiénique pour la miction masculine, selon la revendication 1, **caractérisé en ce que** le tube (2) est de forme approximativement cylindrique.

3. Protecteur hygiénique pour la miction masculine, selon la revendication 1, **caractérisé en ce que** le tube (2) est en forme de cône tronqué.

4. Protecteur hygiénique pour la miction masculine, selon l'une quelconque des revendications précédentes, **caractérisé en ce que** la matière textile est le papier.

5. Protecteur hygiénique pour la miction masculine, selon l'une quelconque des revendications précédentes, **caractérisé en ce que** l'intérieur de la surface du tube (2) incorpore une crème nettoyante pour la peau et les membranes muqueuses.

6. Protecteur hygiénique pour la miction masculine, selon l'une quelconque des revendications ci-dessus, **caractérisé en ce que** la surface interne du tube (2) incorpore une crème désinfectante.
